(19) Europäisches Patentamt
European Patent Office
Office européen des brevets

(11) Veröffentlichungsnummer: **0 017 704**
**B1**

(12) # EUROPÄISCHE PATENTSCHRIFT

(45) Veröffentlichungstag der Patentschrift: **28.07.82**

(51) Int. Cl.³: **C 07 D 279/34,** C 09 B 49/10 // D06P1/30 ,(C07D279/34, 279/20)

(21) Anmeldenummer: **80100408.6**

(22) Anmeldetag: **28.01.80**

(54) **Verfahren zur Herstellung eines Kondensationsproduktes aus Phenothiazin und p-Nitrosophenol und dessen Verwendung zur Herstellung von Schwefelfarbstoffen.**

(30) Priorität: **05.03.79 DE 2908486**

(43) Veröffentlichungstag der Anmeldung: **29.10.80 Patentblatt 80/22**

(45) Bekanntmachung des Hinweises auf die Patenterteilung: **28.07.82 Patentblatt 82/30**

(84) Benannte Vertragsstaaten: **BE CH DE FR GB IT NL**

(56) Entgegenhaltungen:
**EP-A-0 001 801**
**US-A-1 867 863**

**JOURNAL OF THE CHEMICAL SOCIETY, Vol. 125, 1924, London SIKHIBHUSHAN DUTT "Dyes derived from Carbazole and Thiodiphenylamine", Seiten 802-807**

(73) Patentinhaber: **CASSELLA Aktiengesellschaft, Hanauer Landstrasse 526, D-6000 Frankfurt am Main 61 (DE)**

(72) Erfinder: **Nagl, Gert, Dr., Alt-Fechenheim 16, D-6000 Frankfurt am Main 61 (DE)**
Erfinder: **Ribka, Joachim, Dr., Rügener Strasse 4, D-6050 Offenbach Bürgel (DE)**
Erfinder: **Gotsmann, Ulrich, Dr., Speierlingweg 14, D-6000 Frankfurt am Main 60 (DE)**
Erfinder: **Dickmanns, Heinz, Lauterbacher Strasse 8, D-6000 Frankfurt am Main 61 (DE)**

(74) Vertreter: **Urbach, Hans-Georg, Dr. et al, Hanauer Landstrasse 526, D-6000 Frankfurt am Main 61 (DE)**

**EP 0 017 704 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Verfahren zur Herstellung eines Kondensationsproduktes aus Phenothiazin und p-Nitrosophenol und dessen Verwendung zur Herstellung von Schwefelfarbstoffen

Die Erfindung betrifft ein Verfahren zur Herstellung eines mindestens 60 Gew.% des Indophenol-S-Oxyds I

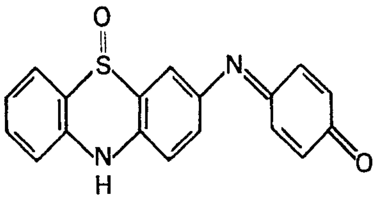

(I)

enthaltenden Kondensationsprodukts aus Phenothiazin und p-Nitrosophenol.

Nach "Journal of the Chemical Society London", Band 125, Seiten 802 und 803 (1924) ist es möglich, Phenothiazin in konzentrierter Schwefelsäure mit p-Nitrosophenol zu kondensieren und das erhaltene Kondensationsprodukt bei 250 bis 300° C mit Schwefel und Natriumsulfid zu grünen Schwefelfarbstoffen zu verschmelzen. Die in dieser Weise hergestellten Schwefelfarbstoffe zeigen jedoch eine Reihe schwerwiegender Nachteile, insbesondere schlechte Ausbeute, geringe Farbstärke, Verunreinigung durch Ausgangs- und Nebenprodukte, unvollständige Löslichkeit in Alkalidithionit und -sulfidküpen, stumpfe Nuancen, ungleichmässigen Ausfall der Produktionschargen, geringe Echtheiten, insbesondere der Nassechtheiten und der Peroxydwaschechtheit, aufgrund deren sie bisher keinen Eingang in die Praxis gefunden haben. Um grüne Schwefelfärbungen auf Baumwolle zu erhalten, war es daher bisher erforderlich, entweder Schwefel enthaltende Derivate des Kupferphthalocyanins einzusetzen, die nicht mit Natriumdithionit verküpbar sind und deren Einsetzbarkeit daher begrenzt und deren Herstellung technisch verhältnismässig aufwendig ist, was ihren Einsatz erheblich verteuert, oder aber auf grüne Schwefelfarbstoffe, wie beispielsweise Sulphur Green 2, C.I. Nr. 53571, oder Sulphur Green 3, C.I. Nr. 53570, zurückgreifen, die jedoch schlechte Nassechtheiten, insbesondere eine schlechte Peroxydwaschechtheit haben und daher nicht für Färbungen mit hohem Echtheitsniveau verwendet werden können. Es bestand daher ein dringendes Bedürfnis nach einem Verfahren, das es gestattet, auf technisch einfache Weise technisch wertvolle Schwefelfarbstoffe herzustellen, die auf Baumwolle echte grüne Färbungen liefern.

Nach einem älteren Vorschlag (deutsche Patentanmeldung P 2748744.1) kann diese Aufgabe gelöst werden, wenn solche Kondensationsprodukte aus Phenothiazin und p-Nitrosophenol der Schwefelung in der Back- oder Kochschmelze unterworfen werden, die unter Mitverwendung eines zusätzlichen Oxydationsmittels erhalten werden und die mindestens 60 Gew.% des Indophenol-S-Oxyds der Formel

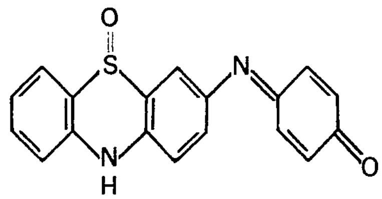

(I)

enthalten. Als zusätzliche Oxydationsmittel können dabei z.B. Schwefeltrioxyd oder überschüssiges p-Nitrosophenol verwendet werden. Bei der Verwendung von p-Nitrosophenol als Kondensations- und als zusätzliches Oxydationsmittel werden dabei mindestens 1,125 mol pro mol Phenothiazin eingesetzt. Die nach dem älteren Vorschlag hergestellten Kondensationsprodukte aus Phenothiazin und p-Nitrosophenol fallen mit hohen Ausbeuten an und sind praktisch frei von Ausgangs- und Nebenprodukten, deren Gegenwart zu einer Qualitätsminderung der aus den Kondensationsprodukten hergestellten Schwefelfarbstoffe führt. Die Schwefelfarbstoffe besitzen hervorragende Echtheiten und eine wertvolle Nuance und können mit hoher Ausbeute, Farbstärke und Reinheit hergestellt werden. Sie sind in Alkalidithionit und Alkalisulfidküpen vollständig löslich.

Es wurde nun gefunden, dass Kondensationsprodukte aus Phenothiazin und p-Nitrosophenol, die mindestens 60 Gew.% des Indophenol-S-Oxyds der Formel I und nur geringe Mengen Ausgangs- und Nebenprodukte enthalten, durch Kondensation von Phenothiazin in Schwefelsäure auch ohne Anwendung eines zusätzlichen Oxydationsmittels erhalten werden können, wenn vor der Zugabe des p-Nitrosophenols das Phenothiazin in 60 bis 90%iger Schwefelsäure, vorzugsweise in 65 bis 88,5%iger Schwefelsäure und ganz besonders bevorzugt in 70 bis 86%iger Schwefelsäure als Radikalkation gelöst vorliegt und dann pro mol mit bis zu 1,125 mol p-Nitrosophenol umgesetzt wird.

Nach der Zugabe des p-Nitrosophenols zu dem in Schwefelsäure gelösten Phenothiazinradikalkation erfolgt die Kondensation zweckmässigerweise bei Temperaturen von −20 bis +20° C, vorzugsweise von −10 bis + 10° C. Die Reaktion ist schwach exotherm, so dass die gewünschte Reaktionstemperatur durch Kühlung eingehalten werden muss. Es ist zweckmässig, einen geringen Überschuss an p-Nitrosophenol anzuwenden. Normalerweise werden pro mol Phenothiazin 1,05 bis 1,125 mol p-Nitrosophenol eingesetzt. Die Isolierung der erhaltenen Kondensationsprodukte erfolgt in üblicher Weise, beispielsweise durch Eintragen des Reaktionsgemischs in Wasser, Abfiltrieren, Auswaschen, Neutralisieren und Trocknen.

Die Konzentrationsangaben für die Schwefelsäure sind in Gewichtsprozenten gemacht und beziehen sich im Rahmen der vorliegenden Erfindung nur auf die Menge der Komponenten Wasser und $H_2SO_4$ in den Lösungen bzw. Reaktionsgemischen. Bei der Berechnung der Schwefelsäurekonzentration ist auch der Schwefelsäureverbrauch durch die Oxydation des Phenothiazins und die Salzbildung sowie die Bildung von Reaktionswasser gemäss folgender Gleichung zu berücksichtigen.

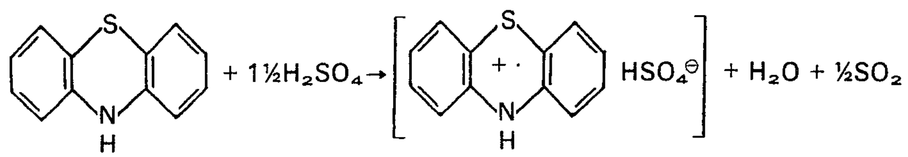


Die Bildung des Phenothiazinradikalkations erfolgt gemäss der vorstehenden Gleichung beim Auflösen von Phenothiazin in Schwefelsäure.

Da sich Phenothiazin in verdünnter Schwefelsäure nur langsam auflöst, ist es zweckmässig, das Phenothiazin zuerst in 90 bis 100%iger Schwefelsäure zu lösen und diese Lösung vor der Kondensation mit p-Nitrosophenol auf eine Schwefelsäurekonzentration von 60 bis 90%, vorzugsweise von 65 bis 88,5%, ganz besonders bevorzugt von 70 bis 86%, zu verdünnen. Für die Durchführung des erfindungsgemässen Verfahrens werden für 1 Gewichtsteil Phenothiazin normalerweise 3,5 bis 10, vorzugsweise 5 bis 8 Gewichtsteile 96 bis 100%ige Schwefelsäure verwendet. Mehr als 10 Gewichtsteile 96 bis 100%ige Schwefelsäure für die Auflösung von einem Gewichtsteil Phenothiazin anzuwenden, ist nicht erforderlich. Wird das Phenothiazin nicht in 96 bis 100%iger Schwefelsäure sondern in verdünnterer Schwefelsäure gelöst, dann ist für die Auflösung entsprechend mehr Schwefelsäure erforderlich. Die erfindungsgemäss hergestellten Kondensationsprodukte enthalten Phenothiazin und Phenothiazin-S-Oxyd zusammen zu weniger als 6 Gew.%, in aller Regel sogar zu weniger als 4 Gew.%, und die Summe der chemisch undefinierten Nebenprodukte liegt unter 20%, in aller Regel sogar unter 10%. Der Hauptbestandteil der erfindungsgemäss hergestellten Kondensationsprodukte ist das Indophenol-S-Oxyd der Formel I mit einem Anteil von 60 bis 100 Gew.%, vorzugsweise 80 bis 100 Gew.%. Ausserdem enthalten die erfindungsgemäss hergestellten Kondensationsprodukte das Indophenol der Formel Ia mit einem Anteil von 0 bis 20 Gew.%, vorzugsweise 0 bis 10 Gew.%.

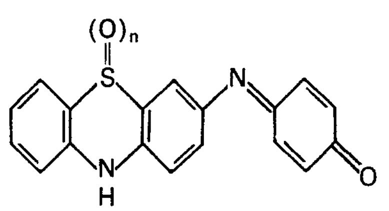

I: n = 1
Ia: n = 0

Die Verbindungen der Formeln I und Ia können auch ganz oder zum Teil in ihren tautomeren Formen vorliegen. Die Ausbeuten des erfindungsgemässen Verfahrens sind gut. Wenn vor der Zugabe des p-Nitrosophenols das Phenothiazin in 70 bis 86%iger Schwefelsäure gelöst vorliegt, wird ein Kondensationsprodukt mit einem Gehalt der Verbindung I von gleich oder grösser als 80 Gew.% in einer Ausbeute von ca. 85% der Theorie oder mehr erhalten. Liegt das Phenothiazin ausserhalb des vorgenannten Bereichs in 65 bis 88,5%iger Schwefelsäure gelöst vor, dann erhält man das Kondensationsprodukt in ca. 80%iger Ausbeute mit einem Prozentgehalt der Verbindung I von 70 oder mehr. Liegt das Phenothiazin ausserhalb der vorgenannten Bereiche in 60 bis 90%iger Schwefelsäure gelöst vor, dann erhält man das Kondensationsprodukt in einer Ausbeute von ca. 75% mit einem Prozentgehalt der Verbindung I von 60 oder mehr.

Die erfindungsgemäss hergestellten Kondensationsprodukte sind hervorragend zur Herstellung von Schwefelfarbstoffen geeignet. Sie werden durch Schwefelung in der Back- oder Kochschmelze in an sich bekannter Weise in Schwefelfarbstoffe überführt, die auf Baumwolle grüne und auf Polyamid grüne bis schwarze Färbungen liefern. Diese Schwefelungsverfahren sind beispielsweise in Venkatarman, "The Chemistry of Synthetic Dyes", Bd. 2 (1952), Seite 1062 ff. und Seite 1103 ff., sowie Bd. 7 (1974), Seite 24 ff., Academic Press, New York, San Francisco, London, beschrieben. Vorzugsweise erfolgt die Schwefelung der erfindungsgemässen Kondensationsprodukte nach dem Verfahren der Kochschmelze. Besonders geeignet sind Kochschmelzen, die unter Verwendung von Alkalipolysulfid arbeiten, wobei pro mol des zu schwefelnden Produkts 2 bis 6 mol, vorzugsweise 2,5 bis 4 mol Natriumsulfid und 7 bis 30 mol, vorzugsweise 13 bis 18 mol Schwefel eingesetzt werden. Sie werden in den für Schwefelschmelzen üblichen Lösungsmitteln durchgeführt. Die aus den nach dem erfindungsgemässen Verfahren hergestellten Kondensationsprodukten herstellbaren Schwefelfarbstoffe besitzen hervorragende Echtheiten und eine wertvolle Nuance und fallen mit hoher Ausbeute, Farbstärke und Reinheit an. Sie sind in Alkalidithionit und Alkalisulfidküpen ausreichend löslich.

In den folgenden Ausführungsbeispielen sind Teile Gewichtsteile. Das Beispiel 1 zeigt die erfindungsgemässe Herstellung des Kondensationsprodukts. Beispiel 2 zeigt zum Vergleich die Ergebnisse, die erhalten werden, wenn nicht nach dem erfindungsgemässen Verfahren gearbeitet wird. Die Beispiele 3 und 5 zeigen die Herstellung von Schwefelfarbstoffen, ausgehend von erfindungsgemäss hergestellten Kondensationsprodukten.

*Beispiel 1*

40 Teile Phenothiazin werden in 250 Teilen konzentrierter (96 gew.%iger) Schwefelsäure unter Kühlung bei Raumtemperatur gelöst. Man kühlt die Lösung auf 0° C und fügt unter Kühlung und kräftigem Rühren 36 Teile Eis zu. Unter Berücksichtigung des Schwefelsäureverbrauchs

durch Oxydation des Phenothiazins und die Salzbildung sowie die Bildung von Reaktionswasser beträgt die berechnete Konzentration der Schwefelsäure nach der Eiszugabe 80,94%. Dann kühlt man die Lösung auf −5° C und fügt unter Kühlung und kräftigem Rühren 30,5 Teile wasserhaltige 85%ige p-Nitrosophenolpaste hinzu. Das Reaktionsgemisch wird 4 h unter Kühlung bei 0° C nachgerührt. Man giesst anschliessend in ein kräftig gerührtes Gemisch aus 1000 Teilen Wasser und 700 Teilen Eis. Das Produkt wird abgesaugt, mit ca. 500 Teilen Wasser gewaschen, mit Wasser angeschlämmt, mit Natronlauge neutralisiert, abgesaugt, salzfrei gewaschen, getrocknet und gemahlen. Man erhält etwa 61 Teile eines Kondensationsprodukts mit einem Gehalt von etwa 88 Gew.% der Verbindung der Formel I und etwa 3 Gew.% der Verbindung der Formel Ia, etwa 2,5 Gew.% Phenothiazin und etwa 0,5 Gew.% Phenothiazin-5-Oxyd (Bestimmung des Gehalts durch HPLC). Ausbeute der Verbindung der Formel I und Ia ca. 87% d.Th.

*Beispiel 2*

40 Teile Phenothiazin werden in 250 Teilen konzentrierter (96 gew.%iger) Schwefelsäure unter Kühlung bei Raumtemperatur gelöst. Unter Berücksichtigung des Schwefelsäureverbrauchs durch Oxydation des Phenothiazins und die Salzbildung sowie die Bildung von Reaktionswasser beträgt die berechnete Konzentration der Schwefelsäure 93,93%. Man kühlt die Lösung auf −5° C und fügt unter Kühlung und kräftigem Rühren 30,5 Teile wasserhaltige 85%ige p-Nitrosophenolpaste hinzu. Das Reaktionsgemisch wird 4 h unter Kühlung bei 0° C nachgerührt. Man giesst anschliessend in ein kräftig gerührtes Gemisch aus 1000 Teilen Wasser und 700 Teilen Eis. Das Produkt wird abgesaugt, mit ca. 500 Teilen Wasser gewaschen, mit Wasser angeschlämmt, mit Natronlauge neutralisiert, abgesaugt, salzfrei gewaschen, getrocknet und gemahlen. Man erhält etwa 59 Teile eines Kondensationsprodukts mit einem Gehalt von etwa 20 Gew.% der Verbindung der Formel I, etwa 30 Gew.% der Verbindung der Formel Ia, etwa 1 Gew.% Phenothiazin und etwa 12 Gew.% Phenothiazin-5-Oxyd (Bestimmung des Gehalts durch HPLC). Der Rest besteht aus undefinierten organischen Verbindungen.

Ausbeute an den Verbindungen der Formeln I und Ia ca. 50% d.Th.

*Beispiel 3*

44 Teile des Kondensationsprodukts von Beispiel 1 werden in eine Polysulfidlösung von etwa 70° C eingebracht, die aus 205 Teilen 95%igem n-Butanol, 49 Teilen schuppenförmigem Natriumsulfid (60%ig) und 58 Teilen Schwefel bereitet wurde. Man erhitzt zum Sieden und hält die Mischung 72 h unter kräftigem Sieden und Rühren am Rückfluss. Man gibt ohne abzukühlen 2,5 Teile Natriumnitrit und 25 Teile 95%iges n-Butanol hinzu und rührt weitere 2 h am Rückfluss. Anschliessend fügt man 300 Teile 24° Bé Salzwasser hinzu und destilliert das Butanol mit Wasserdampf ab. Der Farbstoff wird abgesaugt und mit 12° Bé Salzwasser frei von Polysulfid gewaschen. Der Filterkuchen wird in heissem Wasser angeschlämmt, mit Salzsäure auf pH = 2 gestellt und 1 h bei 60° C nachgerührt. Anschliessend wird abgesaugt, neutral gewaschen, getrocknet und gemahlen. Man erhält etwa 61 Teile Farbstoff in Pulverform. Er färbt die Cellulosefaser aus Alkalisulfid- oder alkalischer Dithionitlösung in grünen Tönen von sehr guter Nass- und Lichtechtheit und Polyamid in grünen bis tiefschwarzen Tönen von sehr guter Nass- und Lichtechtheit.

Der Farbstoff ist in Dithionit-, Sulfid- und Hydrogensulfidküpen praktisch vollständig löslich.

*Beispiel 4* (Vergleichsbeispiel)

Verwendet man in dem Beispiel 3 anstelle des Kondensationsprodukts von Beispiel 1 44 Teile des Kondensationsprodukts von Beispiel 2 und arbeitet man im übrigen nach den Angaben von Beispiel 3, so erhält man etwa 60 Teile Farbstoff in Pulverform. Der Farbstoff unterscheidet sich von dem Farbstoff von Beispiel 3 durch die deutlich geringere Farbstärke, die wesentlich stumpfere und blauere Nuance und die unvollständige Löslichkeit in Dithionit-, Sulfid- und Hydrogensulfidküpen.

*Beispiel 5*

44 Teile des Kondensationsprodukts von Beispiel 1 werden in eine Polysulfidlösung von etwa 70° C eingebracht, die aus 50 Teilen Wasser, 50 Teilen Diäthylenglykolmonoäthyläther, 30 Teilen des Natriumsalzes von m-Xylolsulfonsäure, 49 Teilen schuppenförmigem Natriumsulfid (60%ig) und 58 Teilen Schwefel bereitet wurde. Man erhitzt zum Sieden und hält die Mischung 72 h unter kräftigem Sieden und Rühren am Rückfluss. Die Schmelzmasse wird darauf durch Zugabe von 42 Teilen des Natriumsalzes von m-Xylolsulfonsäure, 53 Teilen schuppenförmigem Natriumsulfid, 160 Teilen einer 30%igen Natriumhydrogensulfidlösung und 250 Teilen Wasser in Lösung gebracht. Man erhält eine wasserdünne, klare, nicht absetzende, haltbare Lösung des reduzierten Schwefelfarbstoffs. Man erhält mit dieser gebrauchsfertigen Farbstofflösung ohne weiteren Zusatz von Reduktionsmitteln auf der Zellulosefaser grüne Färbungen von sehr guter Nass- und Lichtechtheit, auf Polyamidfasern grüne bis tiefschwarze Färbungen von guter Nass- und Lichtechtheit. Der Farbstoff eignet sich in dieser Form besonders gut für die Anwendung nach kontinuierlich arbeitenden Färbeverfahren.

*Beispiel 6* (Vergleichsbeispiel)

Verwendet man in dem Beispiel 5 anstelle des Kondensationsprodukts von Beispiel 1 44 Teile des Kondensationsprodukts von Beispiel 2 und arbeitet man im übringen nach den Angaben von Beispiel 5, so erhält man einen Farbstoff, der sich von dem Farbstoff von Beispiel 5 durch die deutlich geringere Farbstärke, die wesentlich stumpfere und blauere Nuance und die Neigung zum Absetzen unterscheidet.

## Patentansprüche

1. Verfahren zur Herstellung eines Kondensationsprodukts aus Phenothiazin und p-Nitrosophenol, welches das Indophenol-S-Oxyd der Formel I

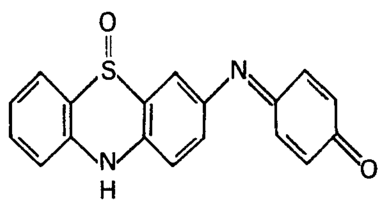

(I)

zu mehr als 60 Gew.% enthält, durch Kondensation von Phenothiazin mit p-Nitrosophenol in Schwefelsäure, dadurch gekennzeichnet, dass das vor der Zugabe des p-Nitrosophenols in 60 bis 90%iger Schwefelsäure als Radikalkation gelöst vorliegende Phenothiazin pro mol mit bis zu 1,125 mol p-Nitrosophenol umgesetzt wird.

2. Verfahren nach Anspruch 1, dadurch gekennzeichnet, dass vor der Zugabe des p-Nitrosophenols das Phenothiazin in 65 bis 88,5%iger Schwefelsäure als Radikalkation gelöst vorliegt.

3. Verfahren nach den Ansprüchen 1 und/oder 2, dadurch gekennzeichnet, dass vor der Zugabe des p-Nitrosophenols das Phenothiazin in 70 bis 86%iger Schwefelsäure als Radikalkation gelöst vorliegt.

4. Verfahren nach einem oder mehreren der Ansprüche 1 bis 3, dadurch gekennzeichnet, dass das Phenothiazin zunächst in 90 bis 100%iger Schwefelsäure gelöst und diese Lösung dann verdünnt wird.

5. Verfahren nach einem oder mehreren der Anspruch 1 bis 4, dadurch gekennzeichnet, dass das als Radikalkation vorliegende Phenothiazin pro mol mit 1,05 bis 1,125 mol p-Nitrosophenol umgesetzt wird.

6. Verwendung der nach einem oder mehreren der Ansprüche 1 bis 5 hergestellten Kondensationsprodukte zur Herstellung von Schwefelfarbstoffen.

## Claims

1. Process of preparing a condensation product of phenothiazine and p-nitrosophenol which contains more than 60% by weight of indophenol-S-oxide of the formula I

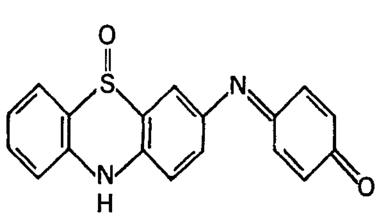

(I)

by condensing phenothiazine with p-nitrosophenol in sulphuric acid, characterized in that the phenothiazine which, prior to the addition of the p-nitrosophenol, is present as a radical cation in solution in 60 to 90% strength sulphuric acid, is reacted, per mol, with no more than 1.125 mol of p-nitrosophenol.

2. The process according to claim 1, characterized in that the phenothiazine is present as a radical cation in solution in 65 to 88.5% strength sulphuric acid before the addition of the p-nitrophenol.

3. The process according to claims 1 and/or 2, characterized in that the phenothiazine is present as a radical cation in solution in 70 to 86% strength sulphuric acid before the addition of the p-nitrosophenol.

4. The process according to one or several of claims 1 to 3, characterized in that the phenothiazine is first dissolved in 90 to 100% strength sulphuric acid and this solution is then diluted.

5. The process according to one or several of claims 1 to 4, characterized in that the phenothiazine which is present as radical cation is reacted, per mol, with 1.05 to 1.125 mol of p-nitrosophenol.

6. Use of the condensation products prepared according to one or several of claims 1 to 5 for the manufacture of sulphur dyestuffs.

## Revendications

1. Procédé de préparation d'un produit de condensation à partir de la phénothiazine et du p-nitrosophénol, dont la teneur en indophénol-S-oxyde de formule I

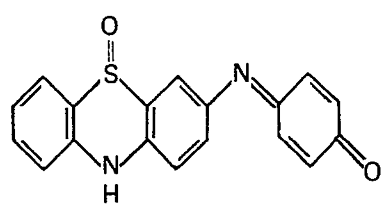

(I)

est supérieure à 60% en poids, par condensation de la phénothiazine avec le p-nitrosophénol dans de l'acide sulfurique, procédé caractérisé en ce qu'on fait réagir la phénothiazine, qui, avant l'addition du p-nitrosophénol, se trouve à l'état dissous sous la forme d'un cation radical dans de l'acide sulfurique d'une concentration de 60 à 90%, avec, par mol, au plus 1,125 mol de p-nitrosophénol.

2. Procédé selon la revendication 1, caractérisé en ce que, avant l'addition du p-nitrosophénol, la phénothiazine se trouve en solution sous la forme d'un cation radical dans un acide sulfurique d'une concentration de 65 à 88,5%.

3. Procédé selon les revendications 1 et/ou 2, caractérisé en ce que, avant l'addition du p-nitrosophénol, la phénothiazine se trouve en solution sous la forme d'un cation radical dans un acide sulfurique d'une concentration de 70 à 86%.

4. Procédé selon une ou plusieurs des revendications 1 à 3, caractérisé en ce qu'on dissout

d'abord la phénothiazine dans un acide sulfurique à 90 à 100%, puis on dilue cette solution.

5. Procédé selon une ou plusieurs des revendications 1 à 4, caractérisé en ce qu'on fait réagir la phénothiazine qui se trouve sous la forme d'un cation radical avec, par mol, de 1,05 à 1,125 mol de p-nitrosophénol.

6. Application des produits de condensation préparés selon une ou plusieurs des revendications 1 à 5, à la préparation de colorants au soufre.